## Europäisches Patentamt

## European Patent Office

## Office européen des brevets

(19)

(11) Veröffentlichungsnummer: **0 085 398**
**B1**

(12) **EUROPÄISCHE PATENTSCHRIFT**

(45) Veröffentlichungstag der Patentschrift:
**27.12.85**

(21) Anmeldenummer: **83100742.2**

(22) Anmeldetag: **27.01.83**

(51) Int. Cl.⁴: **C 07 C  45/49,** C 07 C  47/06,
C 07 C  51/10, C 07 C  53/08,
C 07 C  29/15, C 07 C  31/08,
B 01 J  23/56, B 01 J  27/06,
B 01 J  31/02

(54) Verfahren zur Herstellung von Essigsäure, Acetaldehyd und Ethanol.

(30) Priorität: **30.01.82 DE 3203060**

(43) Veröffentlichungstag der Anmeldung:
**10.08.83 Patentblatt 83/32**

(45) Bekanntmachung des Hinweises auf die Patenterteilung:
**27.12.85 Patentblatt 85/52**

(84) Benannte Vertragsstaaten:
**BE DE FR GB IT NL**

(56) Entgegenhaltungen:
**EP - A - 0 021 241**
**EP - A - 0 022 358**
**US - A - 3 878 290**

**CHEMICAL ABSTRACTS, Band 97, Nr. 9, Oktober 1982,**
**Seite 640, Nr. 144362v, Columbus, Ohio, US**
**CHEMICAL ABSTRACTS, Band 97, Nr. 10, November**
**1982, Seite 660, Nr. 162376g, Columbus, Ohio, US**

(73) Patentinhaber: **HOECHST AKTIENGESELLSCHAFT,**
**Postfach 80 03 20, D-6230 Frankfurt am Main 80 (DE)**

(72) Erfinder: **Leupold, Ernst Ingo, Dr., Am Zäunefeld 15,**
**D-6392 Neu-Anspach (DE)**
Erfinder: **Schmidt, Hans-Joachim, Dr., Am Burgenblick 6,**
**D-6240 Königstein/Taunus (DE)**
Erfinder: **Wunder, Friedrich, Dr., Jahnstrasse 46,**
**D-6093 Flörsheim am Main (DE)**

# 0 085 398

**Beschreibung**

Die vorliegende Erfindung betrifft ein Verfahren zur Herstellung von Essigsäure, Acetaldehyd und Ethanol durch Umsetzung von Kohlenmonoxid mit Wasserstoff an Rhodium und Promotoren enthaltenden Trägerkatalysatoren bei erhöhter Temperaturen und Drücken.

Es ist bekannt, die genannten Verbindungen aus Kohlenmonoxid und Wasserstoff an Rhodium enthaltenden Trägerkatalysatoren herzustellen (DE-AS 2 503 233). Durch Zusatz bestimmter Promotoren bzw. Cokatalysatoren zu den Katalysatoren läßt sich deren Aktivität und/oder deren Selektivität zu den genannten sauerstoffhaltigen Verbindungen beeinflussen. Solche Promotoren sind beispielsweise Magnesium (DE-OS 2 712 732 und DE-OS 2 814 265), Mangan (DE-AS 2 628 463), Eisen (DE-AS 2 503 204) oder eines oder mehrere Elemente der Gruppe Zirkon, Hafnium, Lanthan, Chrom und Quecksilber (DE-OS 2 846 148).

In der EP-A-0 022 358 werden ferner Rhodium-Katalysatoren beschrieben, die als Cokatalysator ein Oxid eines Metalls der Gruppe II a, III a, IV a oder V a des periodischen Systems der Elemente enthalten, beispielsweise Oxide der Seltenen Erden wie Ceroxid, Neodymoxid oder Yttriumoxid. Die Aktivität dieser Katalysatoren ist jedoch gering, sie sind für eine wirtschaftliche Herstellung von Essigsäure, Acetaldehyd und Ethanol wenig geeignet. So reagieren im allgemeinen weniger als 30 Mol-% des umgesetzten Kohlenmonoxids zu Ethanol, der Rest setzt sich hauptsächlich zu Methanol und Kohlenwasserstoffen um. Essigsäure und Acetaldehyd werden an den genannten Katalysatoren nicht oder nur in geringem Maße gebildet.

Aus EP-A-0 021 241 ist eine Synthesegas-Umsetzung bekannt, bei der unter anderem Silikate der seltenen Erden als Träger für Rhodium und Alkalimetalle enthaltende Katalysatoren eingsetzt werden (Seite 4, Zeile 28—35). Eine Imprägnierung von Trägern mit nicht-oxidischen Seltenerden wird jedoch nicht beschrieben. Außerdem entstehen bei der Umsetzung die sauerstoffhaltigen $C_2$-Verbindungen mit wesentlich geringerer Selektivität und Raum-Zeit-Ausbeute als beim Verfahren gemäß vorliegender Erfindung. Aus US-A-3 878 290 ist die Umsetzung von Synthesegas an Katalysatoren bekannt, die neben Rhodium noch Scandium, Yttrium, Cer oder Euopium enthalten können. Bei dieser Umsetzung entstehen jedoch Methanol, Glykole und Glycerin. Weder Ethanol, noch Acetaldehyd oder Essigsäure werden erwähnt.

Es hat sich nun überraschenderweise gezeigt, daß die Aktivität und/oder die Selektivität der Rhodiumkatalysatoren verbessert werden kann, wenn sie als Promoter nichtoxidische Verbindungen von Elementen der Ordnungszahl 21, 39 und 48 bis 71 enthalten.

Gegenstand der Erfindung ist daher ein Verfahren zur Herstellung von Essigsäure, Acetaldehyd und Ethanol durch Umsetzung von Kohlenmonoxid und Wasserstoff an Rhodium und Promotoren enthaltenden Trägerkatalysatoren bei erhöhten Temperaturen und Drücken, das dadurch gekennzeichnet ist, daß als Promotoren ein oder mehrere Elemente der Ordnungszahl 21, 39 und 58 bis 71 in Form nichtoxidischer Verbindungen auf den Träger aufgebracht worden sind.

Bei dem erfindungsgemäßen Verfahren werden Kohlenmonoxid und Wasserstoff in hoher Selektivität zu Essigsäure, Acetaldehyd und Ethanol umgesetzt. Daneben entstehen in geringen Mengen auch solche Produkte, die durch eine Folgereaktion, zum Beispiel durch Veresterung, Acetalisierung oder Kondensation gebildet werden. Hierzu zählen vor allem Ethylacetat und das Diethylacetal des Acetaldehyds. Der Anteil an anderen sauerstoffhaltigen Verbindungen mit drei oder mehr Kohlenstoffatomen im Molekül ist sehr gering und liegt normalerweise unter 5 Mol-%, bezogen auf umgesetzte Kohlenmonoxid. Die Gesamtselektivität zu sauerstoffhaltigen $C_2$-Verbindungen, einschließlich der in Ethylacetat und Acetaldehyddiethylacetal umgewandelten Produkte, beträgt bis zu 82%, bezogen auf umgesetztes Kohlenmonoxid. Der Rest des umgesetzten Kohlenmonoxids wird im wesentlichen in Methan und andere gasförmige Kohlenwasserstoffe und im geringen Maße in Kohlendioxid umgewandelt.

Zum Aufbau des Katalysators für das erfindungsgemäße Verfahren kann man von Salzen oder Komplexverbindungen des Rhodiums ausgehen. Geeignet sind z. B. Chloride, Bromide und Jodide des Rhodiums oder auch Doppelsalze des Rhodiums mit Alkalihalogeniden, wie z. B. Dikaliumtrichlororhodat. Geeignet sind ferner Komplexverbindungen, die neben Rhodium und Halogen noch komplexbildende Liganden, wie Trialkylphosphine, Triarylphosphine, Kohlenmonoxid, Olefine oder Wasser enthalten, also z. B. Tris-triphenylphosphin-rhodium-I-chlorid, -bromid oder -jodid, Tris-triphenylphosphin-rhodium-III-chlorid, Bis-tri-o-tolyl-phosphin-rhodium-II-chlorid, Carbonyl-bis-triphenylphosphin-rhodium-I-bromid oder Dicäsiumcarbonyl-pentachlororhodat-III. Darüber hinaus kommen auch solche Verbindungen des Rhodiums in Betracht, in denen es ionogen oder komplex an einem Träger gebunden ist. Beispiele hierfür sind die mit Rhodiumhalogeniden ausgetauschten Zeolithe und Ionenaustauscher.

Als Promoter wird erfindungsgemäß ein oder mehrere Elemente der Ordnungszahl 21, 39 und 58 bis 71 in Form nichtoxidischer Verbindungen eingesetzt, also beispielsweise die Chloride, Bromide, Fluoride, Nitrate, Acetate, Oxalate, Acetylacetonate oder Tartrate des Scandiums, Yttriums, Cers, Praseodyms, Neodyms, Promethiums, Samariums, Europiums, Gadoliniums, Terbiums, Dysprosiums, Holmiums, Erbiums, Thuliums, Ytterbiums und Lutetiums. Bevorzugt verwendet man die Chloride, Bromide, Acetate und Acetylacetonate dieser Elemente. Besonders bevorzugt sind die Chloride, Bromide,

2

Acetate und Acetylacetonate des Yttriums, Gadoliniums, Dysprosiums, Holmiums und des Ytterbiums, vor allem aber die Chloride dieser Elemente.

Neben den vorstehend genannten Promotoren können die Rhodiumkatalysatoren ggf. noch weitere cokatalytisch wirksame Verbindungen enthalten, beispielsweise Verbindungen des Magnesiums, Lanthans, Mangans, Wolframs, Eisens, ferner Halogenide oder Alkalisalze. Durch Zusatz dieser Verbindungen kann die Leistung und/oder die Selektivität der Katalysatoren ggf. noch verbessert werden.

Als Katalysatorträger werden übliche Trägermaterialien mit unterschiedlichen spezifischen Oberflächen verwendet. Allerdings werden Träger mit spezifischen Oberflächen von 50 bis 1000 m²/g bevorzugt. Geeignet sind z. B. Kieselsäure, natürliche oder synthetische Silikate von Elementen der II. bis VIII. Gruppe des Periodischen Systems (also beispielsweise die Silikate des Magnesiums, Calciums, Aluminiums, Mangans), ferner Aluminiumoxid, Thoriumdioxid, Zeolithe und Spinelle. Vorzugsweise verwendet man Kieselsäure oder Silikate.

Zur Herstellung der Katalysatoren werden die Rhodiumverbindung, die anspruchsgemäßen Promotoren und ggf. weitere Zusatzstoffe, die die Aktivität und/oder die Selektivität der Katalysatoren beeinflussen, gleichzeitig oder in aufeinanderfolgenden Stufen in beliebiger Reihenfolge auf den Träger aufgebracht, und zwar im allg. durch Tränkung des Trägers mit Lösungen der aktiven Komponenten in geeigneten Lösungsmitteln, wie z. B. Wasser, Alkohol, Aceton, Acetylaceton oder Essigsäure und nachfolgendes Trocknen.

Vorzugsweise wird der Katalysator vor Beginn der Synthesegas-Umsetzung noch reduziert. Als Reduktionsmittel kommen beispielsweise Wasserstoff, Kohlenmonoxid, Methanol oder Aceton in Betracht. Diese Reduktion kann in einer getrennten Apparatur oder im Reaktor selbst durchgeführt werden. Vorzugsweise wird bei Temperaturen unter 300°C, insbesondere zwischen 100 und 275°C, reduziert. Vielfach ist es zweckmäßig, die Reduktion nicht mit den unverdünnten reduzierend wirkenden Gasen, sondern mit einem zusätzlichen Anteil an Inertgasen, wie z. B. Stickstoff, Kohlendioxid oder auch Edelgasen, vorzunehmen.

Die Konzentration an Rhodium und Promotor(en) in den Katalysatoren kann in weiten Grenzen varriiert werden; im allgemeinen liegen die Werte zwischen 0,1 und 20 Gew.-% Rhodium und zwischen 0,1 und 25 Gew.-% für die Promotoren. Bevorzugt sind Katalysatoren mit 1,0 bis 10 Gew-% Rhodium und 0,1 bis 20 Gew.-% Promotor(en).

Zur Durchführung des erfindungsgemäßen Verfahrens werden Gasgemische, die ganz oder zu einem überwiegenden Teil aus Kohlenmonoxid und Wasserstoff bestehen und daneben gegebenenfalls noch andere Komponenten wie Stickstoff, Argon, Kohlendioxid oder Methan enthalten können, über den Katalysator geleitet. Das molare Verhältnis von Kohlenmonoxid zu Wasserstoff kann dabei in weiten Grenzen variiert werden. Bevorzugt sind Molverhältnisse zwischen 5 : 1 und 1 : 5 und besonders zwischen 3 : 1 und 1 : 3.

Die Reaktionstemperaturen liegen im allgemeinen zwischen 175 und 400°C, vorzugsweise zwischen 200 und 380°C, und die Reaktionsdrücke zwischen 1 und 300 bar, vorzugsweise zwischen 10 und 200 bar.

Zweckmäßig ist es, Temperatur und Druck so aufeinander abzustimmen, daß eine hohe Selektivität zu den gewünschten Verbindungen gewährleistet ist und und die bei höheren Temperaturen begünstigt exotherme Bildung von Methan gering gehalten wird. Man wird deshalb hohe Drücke und möglichst niedrige Temperaturen bevorzugen. Der Umsatz an Kohlenmonoxid sollte dabei im allgemeinen nicht über 50% liegen, da höhere Umsätze leicht zu vermehrter Nebenproduktbildung führen können, wobei neben Methan, Kohlendioxid und gasförmigen Kohlenwasserstoffen auch höhermolekulare flüssige Kohlenwasserstoffe und sauerstoffhaltige Produkte auftreten können.

Für die Verfahrensdurchführung ist die Gasphase bevorzugt. Hierzu können die herkömmlichen Festbettreaktoren verwendet werden, wobei es zur besseren Wärmeabführung vorteilhaft ist, die Katalysatorschichtdicke gering zu halten.

Ferner sind auch Reaktoren mit bewegtem Katalysatorbett oder Wirbelbettreaktoren geeignet.

Eine besonders bevorzugte Ausführungsform der Erfindung besteht darin, die Umsetzung in einer Kreisgasapparatur in der Gasphase durchzuführen, in der nach Abtrennung der kondensierbaren Reaktionsprodukte das nicht umgesetzte Gasgemisch wieder in den Reaktor zurückgeführt wird.

Diese Verfahrensweise ist besonders wirtschaftlich und ermöglicht durch Verdünnung des Frischgases mit dem im Kreislauf zurückgeführten wasserstoffärmeren Restgas höhere Reaktionstemperaturen und damit höhere Raum-Zeit-Ausbeuten bei unveränderten Selektivitäten. Als Kreisgasapparaturen können dabei solche mit innerem oder äußerem Gasumlauf in Betracht kommen.

Die Erfindung soll durch die folgenden Beispiele erläutert werden, wobei die Beispiele aber in keiner Weise einschränkend sein sollen.

## Beispiele

(»Nl« bedeutet im folgenden »Liter gemessen unter Normalbedingungen«. Die Prozente sind stets Gewichtsprozente).

## I. Allgemeine Versuchsbeschreibung

Die Beispiele werden in einem für Druckreaktionen ausgelegten Rohrreaktor durchgeführt. Es wird mit einem Salzbad beheizt und besteht aus einem Rohr aus rostfreiem Stahl von 16 mm innerem Durchmesser und einem koaxial angebrachten Thermometerrohr von 6 mm äußerem Durchmesser. Der Reaktor ist mit einem Gasvorheizer sowie mit einem nachgeschalteten Kühler zur Kondensation der Reaktionsprodukte und einer Vorlage versehen. Der Reaktor wird mit jeweils 100 ml der im Abschnitt II beschriebenen Katalysatoren gefüllt.

Über den Katalysator leitet man bei den Beispielen 1 bis 11 und den Vergleichsbeispielen 1 bis 3 stündlich 140 NI eines Gemisches von Kohlenmonoxid und Wasserstoff im Volumenverhältnis 1 : 1. Der Reaktionsdruck beträgt 20 bar und die Reaktor-Innentemperatur 310°C. Das Reaktionsgemisch wird gekühlt und die nicht kondensierten Anteile werden entspannt. Die Zusammensetzung von Kondensat und Abgas wird gaschromatographisch bestimmt.

Für die Beispiele 12 bis 16 und die Vergleichsbeispiele 4 und 5 wird die gleiche Apparatur verwendet, jedoch leitet man stündlich 400 NI des Gemisches aus Kohlenmonoxid und Wasserstoff im Volumenverhältnis 1 : 1 bei 70 bar und 330°C über den Katalysator.

## II. Herstellung der Katalysatoren

Es werden je 44 g granulierte Kieselsäure mit einer BET-Oberfläche von 270 m$^2$/g, einer Korngröße von 1,2 bis 3,0 mm, einem Porenvolumen von 1,27 ml/g, einem Schüttgewicht von 0,44 kg/l und einem Natriumgehalt von 0,4% mit einer Lösung der nachstehend aufgeführten Salze in je 50 ml Wasser imprägniert:

|  | Zusatz | g Zusatz pro 50 ml Wasser |
| --- | --- | --- |
| Vergleichsbeispiele 1 und 4 | $MgCl_2 \cdot 6\ H_2O$ | 1,16 g |
| Vergleichsbeispiele 2 und 5 | $LaCl_3$ | 0,25 g |
| Beispiel 1 | $ScCl_3$ | 0,46 g |
| Beispiel 2 | $YCl_3 \cdot 6\ H_2O$ | 0,47 g |
| Beispiel 3 | $CeCl_3$ | 0,24 g |
| Beispiel 4 | $PrCl_3 \cdot 7\ H_2O$ | 0,36 g |
| Beispiel 5 | $NdCl_3 \cdot 6\ H_2O$ | 0,35 g |
| Beispiel 6 | $SmCl_3$ | 0,21 g |
| Beispiel 7 und 13 | $GdCl_3 \cdot 6\ H_2O$ | 0,33 g |
| Beispiel 8 | $TbCl_3$ | 0,23 g |
| Beispiel 9 und 14 | $DyCl_3$ | 0,23 g |
| Beispiel 10 und 15 | $HoCl_3$ | 0,23 g |
| Beispiel 11 und 16 | $YbCl_3 \cdot 6\ H_2O$ | 0,31 g |

Den imprägnierten Träger läßt man 1 Stunde bei Raumtemperatur stehen und trocknet im Trockenschrank unterhalb 80°C.

Der so behandelte Träger wird anschließend mit einer Lösung von 3,62 g $RhCl_3 \cdot x\ H_2O$ (38,0% Rh) in 50 ml Wasser imprägniert und im Trockenschrank unterhalb 80°C getrocknet.

Anschließend wird der Katalysator in einem Strömungsrohr aus Glas durch je 1stündiges Überleiten von 6 NI/h Wasserstoff unter Normaldruck bei 225°C, 250°C und 275°C reduziert.

Der Katalysator für das Vergleichsbeispiel 3 wird wie folgt hergestellt:

170 g $Ce_2O_3$ (100 ml), in Tabletten gepreßt, werden mit einer Lösung von 4,5 g $RhCl_3 \cdot x\ H_2O$

**0 085 398**

(38,1% Rh) in 500 ml Wasser versetzt und anschließend im Rotationsverdampfer und Vakuum bei etwa 100°C bis zur Trockne eingeengt. Der Katalysator wird im Glasgefäß durch je 1stündiges Überleiten von 6 Nl/h Wasserstoff unter Normaldruck bei 225°C, 250°C und 275°C und anschließend im Reaktor durch 15stündiges Überleiten von 10 Nl/h Wasserstoff bei 350°C reduziert.

In der nachfolgenden Tabelle I sind die bei 20 bar und 310°C und in der Tabelle II die bei 70 bar und 340°C erhaltenen Versuchsergebnisse zusammengestellt. Es handerlt sich im um Durchschnittswerte von je 100 Betriebsstunden.

Tabelle 1

Die Katalysatoren enthalten je 3,0% Rh und 0,3% des angegebenen Promotors.
Reaktionsbedingungen: Druck 20 bar, Temperatur 310°C, Raumgeschwindigkeit 1400 h⁻¹.

| | Katalysator-zusammensetzung | RZA[1] $g/l \cdot h$ | Selektivitäten (Mol-% CO)[2] | | | $\Sigma C_2-O$ | $CH_4$ | $C_2-C_4$ Kohlenwasser-stoffe | Sonstige |
|---|---|---|---|---|---|---|---|---|---|
| | | | AcOH | AcH | EtOH | | | | |
| Vergleichsbeispiel 1 | $Rh-Mg-SiO_2$ | 76 | 39,2 | 22,1 | 5,6 | 66,9 | 10,6 | 14,3 | 8,2 |
| Vergleichsbeispiel 2 | $Rh-La-SiO_2$ | 73 | 34,8 | 28,6 | 11,0 | 74,4 | 11,2 | 8,2 | 6,2 |
| Vergleichsbeispiel 3 | $Rh-Ce_2O_3$[3] | 8 | 7,5 | 6,4 | 12,5 | 26,4 | 30,6 | 15,6 | 27,4 |
| Beispiel 1 | $Rh-Sc-SiO_2$ | 83 | 28,2 | 37,6 | 11,4 | 77,2 | 9,4 | 6,5 | 6,9 |
| Beispiel 2 | $Rh-Y-SiO_2$ | 106 | 22,8 | 44,3 | 8,9 | 76,0 | 9,3 | 7,2 | 7,5 |
| Beispiel 3 | $Rh-Ce-SiO_2$ | 76 | 36,7 | 30,0 | 10,5 | 77,2 | 8,7 | 5,2 | 8,0 |
| Beispiel 4 | $Rh-Pr-SiO_2$ | 75 | 34,0 | 31,6 | 12,5 | 78,1 | 7,6 | 6,2 | 8,1 |
| Beispiel 5 | $Rh-Nd-SiO_2$ | 77 | 31,4 | 32,1 | 11,4 | 74,9 | 9,6 | 7,3 | 8,2 |
| Beispiel 6 | $Rh-Sm-SiO_2$ | 75 | 26,8 | 39,5 | 10,8 | 77,1 | 7,8 | 7,5 | 7,6 |
| Beispiel 7 | $Rh-Gd-SiO_2$ | 88 | 25,8 | 41,9 | 9,3 | 77,0 | 8,8 | 7,3 | 6,9 |
| Beispiel 8 | $Rh-Tb-SiO_2$ | 82 | 26,2 | 42,4 | 9,5 | 78,1 | 7,3 | 7,0 | 7,6 |
| Beispiel 9 | $Rh-Dy-SiO_2$ | 89 | 24,9 | 40,0 | 10,3 | 75,2 | 8,9 | 7,8 | 8,1 |
| Beispiel 10 | $Rh-Ho-SiO_2$ | 90 | 25,2 | 46,9 | 10,2 | 82,3 | 5,8 | 6,2 | 5,7 |
| Beispiel 11 | $Rh-Yb-SiO_2$ | 102 | 25,7 | 46,8 | 7,4 | 79,9 | 6,9 | 7,0 | 6,2 |

[1]  RZA = Raum-Zeit-Ausbeute in Gramm sauerstoffhaltige $C_2$-Verbindungen ($C_2-O$ = Essigsäure, Acetaldehyd und Ethanol) pro Liter Katalysator und Stunde
[2]  Molprozent CO bezogen auf umgesetztes Kohlenmonoxid.
[3]  Katalysator im Vergleichsbeispiel 3 enthält 0,95 Gew.-% Rh auf $Ce_2O_3$.

Tabelle II

Die Katalysatoren enthalten je 3,0% Rh und 0,3% Promotor.
Reaktionsbedingungen: Druck 70 bar, Temperatur 330°C, Raumgeschw. 4000 h$^{-1}$.

| | Katalysator-zusammensetzungen | RZA[1]) g/l · h | Selektivitäten (Mol-% CO)[2]) | | | $\Sigma C_2 - O$ | $CH_4$ | $C_2 - C_4$ Kohlen-wasserstoffe | Sonstige |
|---|---|---|---|---|---|---|---|---|---|
| | | | AcOH | AcH | EtOH | | | | |
| Vergleichsbeispiel 4 | $Rh - Mg - SiO_2$ | 348 | 45,2 | 20,5 | 6,5 | 72,2 | 13,2 | 10,6 | 4,0 |
| Vergleichsbeispiel 5 | $Rh - La - SiO_2$ | 364 | 25,9 | 28,5 | 22,4 | 76,8 | 11,5 | 6,0 | 5,7 |
| Beispiel 12 | $Rh - Y - SiO_2$ | 373 | 18,3 | 24,7 | 37,2 | 80,2 | 6,6 | 7,4 | 5,8 |
| Beispiel 13 | $Rh - Gd - SiO_2$ | 392 | 19,8 | 23,5 | 34,8 | 78,1 | 7,8 | 6,9 | 7,2 |
| Beispiel 14 | $Rh - Dy - SiO_2$ | 385 | 18,9 | 34,5 | 27,6 | 81,0 | 6,4 | 5,9 | 6,7 |
| Beispiel 15 | $Rh - Ho - SiO_2$ | 377 | 18,8 | 32,7 | 27,6 | 79,1 | 7,1 | 8,6 | 5,2 |
| Beispiel 16 | $Rh - Yb - SiO_2$ | 443 | 18,5 | 36,7 | 25,3 | 80,5 | 6,9 | 6,2 | 6,4 |

[1]), [2]) vgl. Fußnoten unter Tabelle 1.

0 085 398

**Patentansprüche**

1. Verfahren zur Herstellung von Essigsäure, Acetaldehyd und Ethanol durch Umsetzung von Kohlenmonoxid mit Wasserstoff an Rhodium und Promotoren enthaltenden Trägerkatalysatoren bei erhöhten Temperaturen und Drücken, dadurch gekennzeichnet, daß als Promoter ein oder mehrere Elemente der Ordnungszahl 21, 39 und 58 bis 71 in Form nichtoxidischer Verbindungen auf den Träger aufgebracht worden sind.

2. Verfahren nach Anspruch 1, dadurch gekennzeichnet, daß als nichtoxidische Verbindungen der Promotorelemente die Chloride, Bromide, Acetate oder Acetylacetonate aufgebracht worden sind.

3. Verfahren nach Anspruch 1, dadurch gekennzeichnet, daß als Promotor ein oder mehrere der Elemente Yttrium, Gadolinium, Dysprosium, Holmium, Ytterbium in Form der Chloride, Bromide, Acetate oder Acetylacetonate aufgebracht worden sind.

4. Verfahren nach Anspruch 1, dadurch gekennzeichnet, daß als Promoter ein oder mehrere der Elemente Yttrium, Gadolinium, Dysprosium, Holmium, Ytterbium in Form der Chloride aufgebracht worden sind.

5. Verfahren nach einem der Ansprüche 1 bis 4, dadurch gekennzeichnet, daß als Katalysatorträger Kieselsäure oder Silikate vorhanden sind.

6. Verfahren nach einem der Ansprüche 1 bis 5, dadurch gekennzeichnet, daß der Katalysator neben Rhodium und einem oder mehreren Promotoren zusätzlich Alkalisalze, Magnesium-, Lanthan-, Mangan-, Wolfram- oder Eisenverbindungen und/oder Halogenide enthält.

7. Verfahren nach einem der Ansprüche 1 bis 6, dadurch gekennzeichnet, daß der Katalysator vor Beginn der Umsetzung reduziert worden ist.

**Claims**

1. A process for the manufacture of acetic acid, acetaldehyde and ethanol by reaction of carbon monoxide and hydrogen at elevated temperature and elevated pressure in the presence of carrier catalysts containing rhodium and promotors, characterized in that as promotors one or more elements of the atomic numbers 21, 39 and 58 to 71 in the form of monoxidic compounds have been applied to the carrier.

2. The process as claimed in Claim 1, characterized in that as nonoxidic compounds of the promotor elements the chlorides, bromides, acetates or acetylacetonates have been applied.

3. The process as claimed in Claim 1, characterized in that as promoter one or more of the elements yttrium, gadolinium, dysprosium, holmium, or ytterbium in the form of the chlorides, bromides, acetates or acetylacetonates have been applied.

4. The process as claimed in Claim 1, characterized in that as promoter one or more of the elements yttrium, gadolinium, dysprosium, holmium, ytterbium in the form of the chlorides have been applied.

5. The process as claimed in one of Claims 1 to 4, characterized in that as catalyst carrier silicic acid or silicates are present.

6. The process as claimed in one of Claims 1 to 5, characterized in that the catalyst contains alkali salts, magnesium, lanthanum, manganese, tungsten or iron compounds and/or halides in addition to rhodium and one or more promoters.

7. The process as claimed in one of Claims 1 to 6, characterized in that the catalyst has been reduced before starting the reaction.

**Revendications**

1. procédé pour la préparation d'acide acétique, d'acétaldéhyde et d'éthanol par réaction de monoxyde de carbone sur de l'hydrogène, sur des catalyseurs supportés contenant du rhodium et des promoteurs, à températures et pressions élevées, caractérisé en ce qu'on applique sur le support, en tant que promoteur, un ou plusieurs éléments de numéros atomiques 21, 39 et 58 à 71 sous la forme de composés autres que leurs oxydes.

2. Procédé selon la revendication 1, caractérisé en ce qu'on applique en tant que composés des éléments promoteurs autres que leurs oxydes les chlorures, bromures, acétates ou acétylacétonates.

3. Procédé selon la revendication 1, caractérisé en ce qu'on applique en tant que promoteur un ou plusieurs des éléments yttrium, gadolinium, dysprosium, holmium, ytterbium, sous la forme de leurs chlorures, bromures, acétates ou acétylacétonates.

4. Procédé selon la revendication 1, caractérisé en ce qu'on applique en tant que promoteur un ou plusieurs des éléments yttrium, gadolinium, dysprosium, holmium, ytterbium, sous la forme de leurs chlorure.

5. Procédé selon l'une des revendications 1 à 4, caractérisé en ce que le support du catalyseur est constitué de silice ou de silicates.

6. Procédé selon l'une des revendications 1 à 5, caractérisé en ce que le catalyseur contient, outre le rhodium et un ou plusieurs promoteurs, des sels de métaux alcalins, des composés du magnésium, du lanthane, du manganèse, du tungstène ou du fer, et/ou des halogénures.

7. Procédé selon l'une des revendications 1 à 6, caractérisé en ce que le catalyseur a été réduit avant le début de le réaction.